# EUROPEAN PATENT APPLICATION

(11) **EP 2 827 310 A1**
(43) Date of publication of application: **21.01.2015**
(21) Application number: 13176591.9
(22) Date of filing: 16.07.2013
(51) Int. Cl.: G08B 25/01, A61B 5/00, G06F 19/00

(54) **System for launching an alarm**

(71) Applicant: Televic Healthcare NV, 8870 Izegem (BE)
(72) Inventor: Verheye, Marino, 8760 Meulebeke (BE); Verhaeghe, Geert, 8680 Koekelare (BE)
(74) Representative: Van Bladel, Marc

(57) **Abstract**

The present invention relates to a system for launching an alarm in a healthcare environment comprising
- an object (1,2) comprising means for generating a signal and comprising an output for transmitting the generated signal and
- a terminal (30) arranged for receiving the generated signal and for launching an alarm or for generating an event,
characterised in that the object is squeezable and in that, when the object is squeezed, energy is produced for generating the signal so that a patient can activate the system by pushing or throwing said object.

## Description

### Field of the invention

The present invention is generally related to the field of wireless devices and systems for launching an alarm in a healthcare environment.

### Background of the invention

For applications in a healthcare environment it is of great importance that alarm systems are robust and easy-to-use, for e.g. elderly people and little children as well as for mentally disabled patients.

Such persons may be unable, due to age, handicap, technical skills or mental state to activate or a push a button that generates an alarm. After the alarm signal is launched, it is subsequently handled by a central system, which notifies a care taker that a patient or resident needs help. As this alarm cannot be missed, an alternative solution is needed instead of a standard alarm button.

In application US2012/108299 a vibration module is disclosed for a portable terminal. When vibrating the vibration module generates sufficient vibration power through acceleration at the instant of changing moving direction at the ends of a moving section of the terminal, to provide an alarm function, such as an incoming call notification. The vibration module has a short response time, provides a more effective click feeling and haptic feedback function even during time of rapid, continuous key input operation via a touch screen. The vibration module can generate various haptic patterns corresponding to touch screen operations, such as drag, as well as providing a click feeling similar to a button click feeling at the time of key input through a touch screen.

International application WO2012/136157 relates to a wireless bedwetting alarm and a disposable diaper product comprising the alarm. The alarm comprises a sensor component, an RFID reader and an alarm terminal. The sensor component is a passive RFID tag or a moisture sensor. The sensor obviates the need for a standalone power source apparatus for power supply. Power is supplied by receiving and converting into electricity the energy wirelessly emitted by the RFID reader or by harvesting body energy, temperature, or movement of the body of a user. The RFID reader uses a wireless mode for transmitting a signal to the alarm terminal to issue an alarm. The low cost solution as proposed employs passive technology and obviates the use of battery.

Hence, there is a need for a solution wherein a wireless alarm device that can be integrated in the patient environment and that allows generating an alarm signal in a simple way.

### Summary of the invention

It is an object of embodiments of the present invention to provide for a communication system for launching an alarm that is very simple to use by any patient.

The above objective is accomplished by the solution according to the present invention.

In a first aspect the invention relates to a system for launching an alarm in a healthcare environment comprising
- an object comprising means for generating a signal and comprising an output for transmitting the generated signal and
- a terminal arranged for receiving said generated signal and for launching an alarm or for generating an event,
**characterised in that** the object is squeezable and in that, when the object is squeezed, energy is produced for generating the signal so that a patient can activate the system by pushing or throwing the object.

The proposed system indeed is very straightforward in its use. By pushing or throwing the squeezable object a signal is generated by exploiting energy produced when the object is squeezed. Via an output of the object the generated signal is wirelessly transmitted to a terminal. The terminal then processes that signal and launches the alarm or generates an event. An important advantage of the proposed solution is that the patient is not bothered by technical problems, like low battery. The wireless object only relies on the energy produced when squeezing. In certain embodiments the object has no energy storage inside. Another important asset of the proposed system is its user friendliness. It is suitable for use by virtually any patient, ranging from young children to elderly people as well as patients whose disease or mental state is so that e.g. clicking a button is too difficult.

Various options exist to produce energy when the object is squeezed. In a preferred embodiment the system is adapted for producing the energy by compressing air. The squeezable object then typically comprises a valve for outputting the generated signal.

In another preferred embodiment the system comprises an energy harvester arranged for collecting the energy produced when the object is squeezed, energy conversion means for conversion into electrical energy and processing means adapted for generating the signal. Advantageously, the system of the invention then further comprises energy storage means for storing the electrical energy.

In a preferred embodiment the system further comprises a transducer for transducing a processing means output into the signal to be transmitted via the output.

The means for generating are preferably adapted for generating a plurality of signals. Advantageously the signals of said plurality of signals each have a different frequency. In an embodiment of the invention each signal of said plurality has an own output.

For purposes of summarizing the invention and the advantages achieved over the prior art, certain objects and advantages of the invention have been described herein above. Of course, it is to be understood that not necessarily all such objects or advantages may be achieved in accordance with any particular embodiment of the invention. Thus, for example, those skilled in the art will recognize that the invention may be embodied or carried out in a manner that achieves or optimizes one advantage or group of advantages as taught herein without necessarily achieving other objects or advantages as may be taught or suggested herein.

The above and other aspects of the invention will be apparent from and elucidated with reference to the embodiment(s) described hereinafter.

### Brief description of the drawings

The invention will now be described further, by way of example, with reference to the accompanying drawings, wherein like reference numerals refer to like elements in the various figures.

Fig.1 illustrates an embodiment of the squeezable object of the system according to the invention with a valve.

Fig.2 illustrates an embodiment of the squeezable object comprising an electronic circuit and energy harvesting means.

Fig.3 illustrates a block scheme of a possible implementation of the electronic circuit shown in Fig.2.

Fig.4 illustrates an embodiment of the terminal device of the system according to the invention.

Fig.5 illustrates a first exemplary use case.

Fig.6 represents a second exemplary use case.

### Detailed description of illustrative embodiments

The present invention will be described with respect to particular embodiments and with reference to certain drawings but the invention is not limited thereto but only by the claims.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

It should be noted that the use of particular terminology when describing certain features or aspects of the invention should not be taken to imply that the terminology is being redefined herein to be restricted to include any specific characteristics of the features or aspects of the invention with which that terminology is associated.

In the description provided herein, numerous specific details are set forth. However, it is understood that embodiments of the invention may be practiced without these specific details. In other instances, well-known methods, structures and techniques have not been shown in detail in order not to obscure an understanding of this description.

The present invention discloses a communication system that is primarily intended for use in a healthcare environment. The system is so designed that an alarm is launched from a terminal when this terminal has received and processed a signal transmitted from a squeezable object that has been pushed by a patient. Hence, the alarm is indirectly launched with a wireless tag or device (object).

An important feature of the object generating a signal when it is squeezed, is that it contains no battery. Hence, the object has a long lifetime.

A number of embodiments of the system according to the invention is now described with more detail.

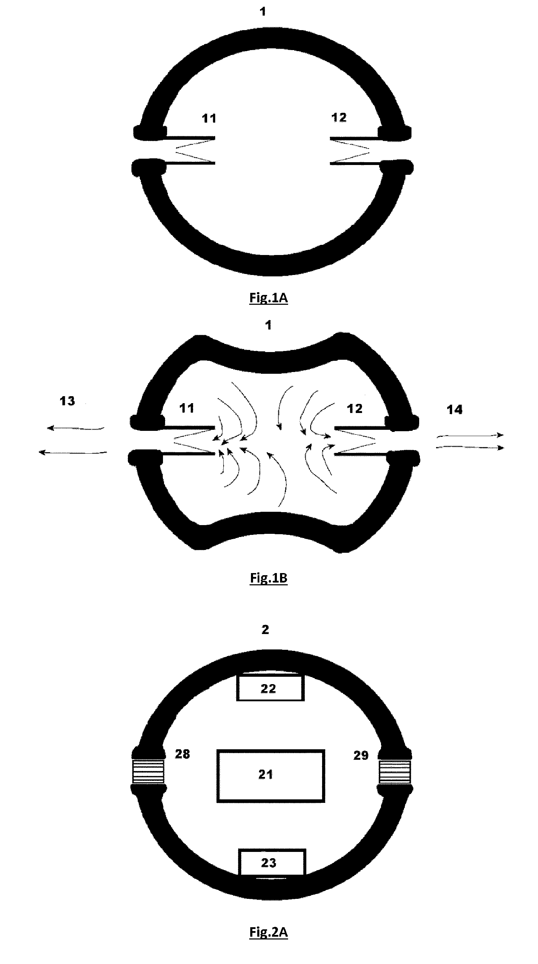

First a system implementation is presented with a valve. Fig.1 illustrates an object (1) with two valves (11,12). The object in the figure has the shape of a ball. In Fig.1A the ball is in an inactive state. In Fig.1B the ball (1) is squeezed and by air compression two signals (13,14) are generated. Advantageously, the generated signals have a different frequency.

In one embodiment the signals are either ultrasound or audible sound signals. Whereas audible sound is approximately between 15 Hz and 20 kHz, ultrasound signals have a frequency above the limit of the human hearing, approximately above 20 kHz. The valve shape is adapted for this purpose. The valve contains a cavity or hollow confined space. The air stream is split by a bevel, and partly whirls around the cavity before exiting through an opening (or sound hole) which is usually small in proportion to the size of the cavity. The size of the whistle cavity and the volume of air contained in the whistle determine the pitch or frequency of the sound produced. Hence, if the air is passing the lip of the valve, the lip inside the valve starts to vibrate and produces the requested sound frequency.

In another embodiment only one output signal is generated.

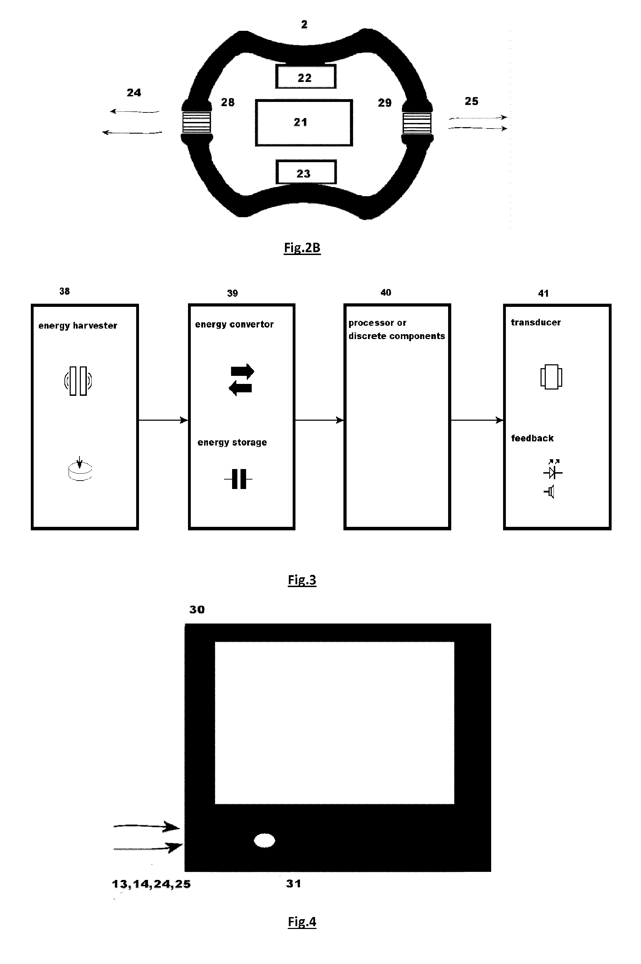

Fig.2 illustrates another embodiment of the invention. An object (2) of the same shape as in Fig.1 is shown. It contains an electronic circuit (21) and two energy harvesters (22,23). On the left and right hand side of the figure outputs (24,25) provided with a transducer (28,29) are shown. Fig.2A again shows the inactive state, where in Fig.2B the active state is shown. When the ball (2), e.g. a gummy ball, is pushed or squeezed, the energy harvesters generate power for the electronic circuit (21). In the electronic circuit the energy is converted.

Fig.3 illustrates a block scheme of a possible implementation of the electronic circuit (21). Because the gummy ball does not contain any 'long time' energy storage like a conventional or rechargeable battery, the energy has to be obtained in another way in the embodiment shown in Fig.3. The principle of energy harvesting is applied. Whenever the ball is pushed or squeezed, the harvester component (22,23) transfers the energy coming from the push or squeeze to electrical energy, that can be used to power the electronic circuit (21).

The energy gathered form the energy harvester needs to be converted in an energy converter (26) to a correct voltage adapted for the low-power electronic circuit. Because the released energy is only available for a short period of time, this energy needs to be stored in a storage capacitor. This capacitor receives a higher voltage, but short energy peak and converts it toward lower power voltage that can be used for a longer time.

The processing means (27) in Fig.3 is implemented as a single processor or as set of interconnected discrete components. This block is arranged to control the transducer (28,29) so that a signal can be transmitted to the detection device. The particular configuration of the processor or the composition of discrete components defines how long the transducer will be activated or what the sequence the generated frequency will be. Secondly this block controls the connected notification block. It can be visual or audible feedback for the user who pushed or squeezed the ball. In this way the user will hear or see feedback and is assured about his action. The particular configuration of the processor or the composition of discrete components defines how long the signal will last.

Block 28 in Fig.3 represents the transducer and notification block. The transducer contains an electrical component that generates a signal detectable by a detection device in the receiving terminal. This transducer is mounted in that way, it can send the signal on a direct way out of the ball, for example via a hole or gap in the shell of the ball. The notification block is needed to notify the user of his action, so the user has a feedback and he can be sure that the alarm is sent to the receive device. The notification can be a visual LED or an audible sound.

In an alternative implementation of output block 28, one can have two transducers, which each have different characteristics of their own (e.g. different frequencies).

In an advantageous embodiment the two frequencies are used. This can serve different purposes :
- security : to eliminate processing of an ultrasound signal generated by accident or transmitted by another, third-party device,
- variation : depending on the two frequencies, one can create variation in the functionality and make multiple types of the gummy ball and apply a different processing on the received signals. To optimize the quality of the transmitted signal we can use multiple holes or gaps in the shell of the ball.

Fig.4 shows an embodiment of the terminal device (30). The terminal device comprises a signal receiver (31). This receiver is adapted for receiving the signals (13,14,24,25) from the ball (2). When the signal is validated (taking into account e.g. the signal frequency or the time duration of the signal) and the alarm is localized in the room where the terminal is mounted, the terminal device sends the alarm information to a central control system, where it can be processed and monitored. The information sent to the central control system comprises one or more parameters, like e.g. the physical location of the terminal where the signal is received, the type of alarm (patient call, nurse call, ...), an identification of the gummy ball that was used, an indication of the location of the alarm (near the toilet, near the door, ...).

Based on the received parameters the central control system can decide what action must be taken to notify the nurses that patient needs help. The outcome of the processing in the central control system can be any action that can help the person who activated the alarm on the gummy ball. The actions depends the configuration of the central system. A few dependencies are given below
- Time of the day (in the morning, at noon, between 18:00 and 19:00 h. etc. ...)
- The day (each Monday, in the weekend, first day of the month, at one specific day of the year)
- The ward or department the terminal is located, or the type of ward or department
- The room where the alarm is launched
- The location, occupation or schedule of the care givers
- The priority of the alarm (urgent, normal ...)
- The needs or desires of the patient (age of the patient, mental state...)
- If a nurse is already in the room or nearby the patient

Some examples of actions that can be triggered on reception of the alarm signal are the following.
- A nurse can be notified on her smartphone that the user who initiated the action needs help in the room where the alarm is detected.
- A signage board shows the alarm with information on the user and the room of the actions.
- The alarm is displayed on other terminals of the same central system. A lamp or LED light is activated near the room where the action has been taken.
- Whenever the gummy ball is pushed or squeezed, an intercom voice call is started so the patient can speak to a helpdesk, than the care giver and patient can start a conversation,
- An audible sound is activated on a buzzer or loudspeaker on a specific rhythm or tone.
- A notification e-mail or text message is sent.
- The alarm of the user is saved in logbook database, so that one can check the action history of all actions taken.
- All those actions can be sent to different stakeholders of the system (Nurse, Doctor, Family, Staff, etc...)
- Depending different time intervals, the action to be taken can be different. For example, at night another nurse is notified as at daytime.

In the examples above the patient's alarm is used to notify a care giver. However, the launched alarm signal can have more functionalities and generate other events. One illustration is that when the gummy ball is pushed or squeezed, it will activate or de-activate the radio of television.

Advantageously the squeezable object (e.g. a gummy ball) is so designed that it can generate at least two signals, preferably at different frequencies. The use of two or more (specific) frequencies adds security to the communication : it avoids processing a signal generated by accident or transmitted by another, third-party device. Further the use of multiple frequencies allows for variation : dependent on the two frequencies, one can make variation in the functionality and make multiple types of the gummy ball and attach different processes on the received signals.

The process that can be started by the different gummy balls can vary according to the configuration of the healthcare system. As an example two use cases are described below.

### Example 1 : a patient mentally not able to click a button is calling a care giver

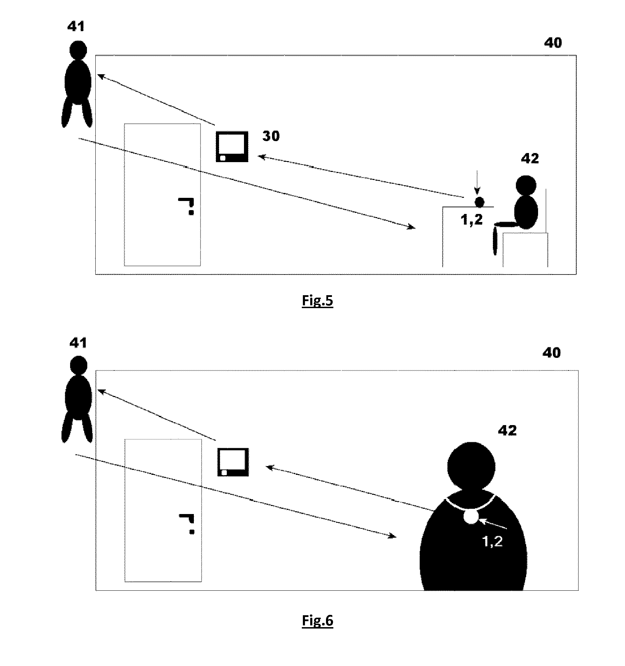

Reference is made to Fig.5. This example describes a patient's room (40), an area surrounded by walls wherein a patient 42 is located. The patient wants to launch an alarm so that a nurse is notified about the patient's call for help. It is assumed the patient is not able to click a button e.g. because of his mental state. Nevertheless he or she should be able to launch an alarm. However, the patient can still be taught some basic actions how to call for help. It is still possible to explain to the patient that they should push or squeeze this object, if they need help. In phase X the patient pushes or hits the gummy ball. Whenever the patient needs help, he or she can push or squeeze the gummy ball. The only requirement is that the ball be near to the patient, so that he can squeeze it. In this example the gummy ball is lying on the table, but it can also be in another area of the room, for example in the chair, in a closet, near the door, on a closet, etc...

The gummy ball sends one or more ultrasound signals to the receiver device.

In phase 5 the receiver processes the signal. The receiver device will thereby validate, localize and process the signal. This information is conveyed to the central system along with the alarm information and the localization information. Next a care giver is alarmed by the healthcare system. The care giver is notified by the central system. The type of notification can be anything; mostly it is a call to a smartphone, a text message or an output lamp.

The care giver 41 will help the patient. When the care giver receives or sees this notification, the patient can be helped.

### Example 2 : a patient not able to change technical objects is calling a care giver

Reference is made to Fig.6. This example describes a patient's room, an area surrounded by walls wherein a patient is located. This patient wants to launch an alarm so that a nurse is notified about the patient's call for help.

Although the patient is not able to change technical objects, due to a lack of technical skills, he or she should still be able to launch an alarm, without having to bother if the battery is low or this alarm device needs electrical power.

Whenever the patient needs help, he or she can push or squeeze the gummy ball. The only requirement is that this ball be near to the patient, so that he can squeeze it. In this example the gummy ball is part of a necklace.

The gummy ball sends ultrasound to the receiver. When it is pushed, the gummy ball is sending a signal or signals to the receiver device. The receiver processes the signal. The receiver device will validate, localize and process the signal. This information is with the alarm information and the localization information to the central system.

A care giver 41 is alarmed by the healthcare system. The care giver is notified by the central system. The notification can be of any type. Mostly it is a call to a smartphone, a text message or an output lamp. The care giver will help the patient. As soon as the care giver receives or sees this notification, the patient can be helped.

Although the invention is described as a gummy ball, the shape factor can be different from a (perfect) ball. This invention can also be used in squeezable squares, triangles or any other shapes can be used for this purpose (a shape in form of a house, a dog, a doll, a watch etc....) as long as it does not stigmatise the patient not able to launch an alarm in a standard way.

While the invention has been illustrated and described in detail in the drawings and foregoing description, such illustration and description are to be considered illustrative or exemplary and not restrictive. The foregoing description details certain embodiments of the invention. It will be appreciated, however, that no matter how detailed the foregoing appears in text, the invention may be practiced in many ways. The invention is not limited to the disclosed embodiments.

Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. System for launching an alarm in a healthcare environment comprising
- an object (1,2) comprising means for generating a signal and comprising an output for transmitting the generated signal and
- a terminal (30) arranged for receiving said generated signal and for launching an alarm or for generating an event,
**characterised in that** said object is squeezable and **in that**, when the object is squeezed, energy is produced for generating said signal so that a patient can activate said system by pushing or throwing said object.

2. System for launching an alarm as in claim 1, adapted for producing said energy by compressing air, said squeezable object comprising a valve (11,12) for outputting said generated signal.

3. System for launching an alarm as in claim 1, comprising an energy harvester (22) arranged for collecting said energy produced when the object is squeezed, energy conversion means for conversion into electrical energy and processing means adapted for generating said signal.

4. System for launching an alarm as in claim 3, further comprising energy storage means for storing said electrical energy.

5. System for launching an alarm as in claim 3 or 4, further comprising a transducer for transducing a processing means output into the signal to be transmitted via said output.

6. System for launching an alarm as in any of the previous claims, wherein said means for generating are adapted for generating a plurality of signals.

7. System for launching an alarm as in claim 6, wherein the signals of said plurality of signals each have a different frequency.

8. System for launching an alarm as in claim 6 or 7, wherein each signal of said plurality has an own output.

9. System for launching an alarm as in any of the previous claims, wherein said generated signal has a frequency in the ultrasound range.
